# FASCICULE DE BREVET EUROPEEN

(11) **EP 0 485 578 B1**
(45) Date de publication et mention de la délivrance du brevet: **02.10.1996**
(21) Numéro de dépôt: 91910774.8
(22) Date de dépôt: 05.06.1991
(51) Int. Cl.: C12N 15/12, C07K 14/475, A61K 38/18, C12N 5/02, A61K 48/00, A61K 35/30

(54) **FACTEURS DE CROISSANCE NEUROTROPES COMPRENANT UN PEPTIDE HOMEOBOITE**
NEUROTROPISCHE WACHSTUMSFAKTOREN, DIE EIN HOMEOBOX-PEPTID ENTHALTEN
NEUROTROPIC GROWTH FACTORS COMPRISING A HOMEOBOX PEPTIDE

(30) Priorité: 05.06.1990 FR 9006912
(43) Date de publication de la demande: 20.05.1992
(73) Titulaire: CENTRE NATIONAL DE LA RECHERCHE SCIENTIFIQUE (CNRS), 75794 Paris Cédex 16 (FR)
(72) Inventeur: JOLIOT, Alain, F-75012 Paris (FR); PROCHIANTZ, Alain, F-75005 Paris (FR)
(74) Mandataire: Ores, Irène
(86) Numéro de dépôt international: FR9100444
(87) Numéro de publication internationale: WO9118981

(56) Documents cités:
- WO-A-90/06757
- Proc. Natl. Acad. Sci., volume 86, juin 1989, Neurobiology, P. Ernfors et al.: "A cell line producing recombinant nerve growth factor evokes growth responses in intrinsic and grafted central cholinergic neurons", pages 4756-4760, voir l'article en entier (cité dans la demande)
- Nature, volume 318, 19/26 décembre 1985; C. Desplan et al.: "The drosophila developmental gene, engrailed, encodes a sequence-specific DNA binding activity", pages 630-635, voir l'article en entier
- Proc. Natl. Acad. Sci. USA, volume 88, no. 5, mars 1991, Neurobiology; A. Joliot et al.: "Antennapedia homeobox peptide regulates neural morphogenesis", pages 1864-1868, voir l'article en entier
- The EMBO Journal, volume 7, no. 13, 20 décembre 1988; M. MÜLLER et al.: "Isolation and sequence-specific DNA binding of the antennapedia homeodomain", pages 4299-4304, voir l'article en entier (cité dans la demande)
- The EMBO Journal, volume 7, no. 13, 20 décembre 1988, IRL Press Limited (GB); G. Otting et al.: "Secondary structure determination for the antennapedia homeodomain by nuclear magnetic resonance and evidence for a helix-turn-helix motif", pages 4305-4309, voir l'article en entier (cité dans la demande)
- Nucleic Acids Research, volume 17, 1989, IRL Press; D. Acampora et al.: "The human HOX gene family", pages 10385-10402, voir l'article en entier (cité dans la demande)
- Cell, volume 55, 18 novembre 1988, Cell Press; M. Levine et al.: "Homeobox proteins as sequence-specific transcription factors", pages 537-540, voir l'article en entier
- The EMBO Journal, volume 7, no. 7, 1988, IRL Press Ltd, (GB); K.W.Y. Cho et al.: "Differential utilization of the same reading frame in a xenopus homeobox gene encodes two related proteins sharing the same DNA-binding specificity", pages 2139-2149, voir l'article en entier, en particulier figure 5 et page 2144, colonne 1
- Proc. Natl. Acad. Sci. USA, volume 83, décembre 1986, Developmental Biology; A. Fainsod et al.: "The homeo domain of a murine protein binds 5' to its own homeo box", pages 9532-9536, voir l'article en entier
- Bio Essays, volume 10, nos. 2,3, février-mars 1989; D.G. Wilkinson: "Homeobox genes and development of the vertebrate CNS", pages 82-85, voir l'article en entier
- Mol. Cell. Biol., volume 6, no. 12, décembre 1986, American Society for Microbiology; A. Laughon et al.: "Structure of transcripts from the homeotic antennapedia gene of drosophila melanogaster: Two promoters control the major protein-coding region", pages 4676-4689, voir l'article en entier, en particulier figure 8

## Description

La présente Invention est relative à l'utilisation des peptides homéoboîtes, ou de peptides dérivés de ceux-ci, pour l'obtention de médicaments.

On désigne sous le nom de peptides homéoboîtes une famille de séquences peptidiques apparentées, que l'on retrouve chez différentes espèces animales dans les produits de gènes impliqués dans l'embryogenèse.

On connaît en effet, des gènes qui s'expriment à différents stades du développement embryonnaire, et dont l'expression contrôle les phénomènes de migration et différenciation cellulaires impliqués dans la morphogénèse de l'organisme.

Ces gènes sont appelés gènes homéotiques et leurs produits de traduction sont appelés homéoprotéines.

L'un de ces gènes qui a été tout particulièrement étudié, est le gène Antennapedia de la Drosophile ; l'analyse de ce gène a permis de mettre en évidence une séquence d'ADN d'environ 180 pb, baptisée séquence homéoboîte.

Cette séquence homéoboîte présente la particularité d'être hautement conservée dans de nombreux gènes homéotiques, et ce, non seulement chez la drosophile, mais également au cours de l'évolution, chez différentes espèces animales. Des séquences homéoboîtes homologues de celle de la Drosophile ont ainsi été trouvées chez tous les vertébrés, y compris les mammifères [ACAMPORA et al., NUCLEIC ACID RES., 17, 10385, (1989)].

La séquence homéoboîte code pour une séquence polypeptidique de 60 aminoacides, qui correspond à une région structurellement et fonctionnellement conservée présente dans toutes les homéoprotéines, l'homéodomaine. La séquence de l'homéodomaine codé par la séquence homéoboîte du gène Antennapedia est indiquée ci-après à titre d'exemple.

(I)
NH2Arg Lys Arg Gly Arg Gln Thr Tyr Thr Arg Tyr Gln Thr
Leu Glu Leu Glu Lys Glu Phe His Phe Asn Arg Tyr Leu Thr
Arg Arg Arg Arg Ile Glu Ile Ala His Ala Leu Cys Leu Thr
Glu Arg Gln Ile Lys Ile Trp Phe Gln Asn Arg Arg Met Lys
Trp Lys Lys Glu AsnCOOH.

Le rôle et le mécanisme d'action de la séquence homéodomaine ont fait l'objet de diverses recherches. On sait ainsi actuellement que cette séquence permet la fixation des homéoprotéines à l'ADN, au niveau de séquences consensus incluant le motif ATTA, présentes dans les promoteurs ou les séquences d'amplification de différents gènes, y compris les gènes à homéoboîte eux-mêmes.

MULLER et al [EMBO J., 7, 4299,(1988)] ont cloné la séquence homéoboîte d'Antennapedia, et purifié le polypeptide correspondant, ou peptide homéoboîte (pAntp). En présence d'un agent réducteur, ils sont obtenu le polypeptide sous forme d'un monomère, de coefficient de sédimentation d'environ 1 S, et de poids moléculaire apparent 9040 Da (Poids moléculaire théorique, d'après la séquence peptidique = 8545 Da).

En l'absence d'agent réducteur, la préparation de polypeptide contenait une importante proportion de dimères, correspondant à des homéodomaines liés entre eux par des ponts disulfure

Ces mêmes auteurs ont également montré que le polypeptide purifié sous forme monomérique se liait à l'ADN, au niveau d'une séquence ANNNNCATTA, contenant donc la séquence consensus ATTA.

Etant donné le très haut degré de conservation des séquences homéoboîtes d'une espèce à une autre, il est considéré que les propriétés du peptide pAntp peuvent être généralisées aux autres peptides homéoboîte, pouvant différer dans leur séquence par quelques acides aminés, mais dotés de propriétés fonctionnelles quasi-identiques ; dans ce qui suit, les termes "peptide homéoboîte" désignera indifféremment aussi bien le peptide pAntp que tout autre membre de ladite famille, ou d'une famille proche, par exemple la famille "engrailed".

D'autres travaux [OTTING et al, EMBO J., 7, 4305, (1988)] ont établi que l'homéodomaine possédait une structure particulière (hélice/tour β/hélice), qui serait impliquée dans la liaison à l'ADN.

La liaison peptide homéboîte/ADN serait la résultante :
- d'une liaison à haute affinité (K_{D≈} 10⁻⁹-10^{-10M}), impliquant la séquence consensus ATTA,
- et d'une liaison à faible affinité (K_{D≈} 10⁻⁶-10^{-7M}), impliquant le sillon large de la double hélice d'ADN. Une publication de KISSINGER et al. [Cell, 63, 579-590, (1990)] décrit une étude faite par cristallographie sur un complexe "homéodomaine engrailed/ADN". Cette étude montre que la partie C terminale de l'homéodomaine, comprenant en particulier la structure dénommée hélice 3 (acides aminés 42-58 de l'homéodomaine engrailed), se fixe au niveau du sillon large de l'ADN. La liaison est essentiellement assurée par des interactions hydrophiles ; cette liaison serait indépendante de la présence de la séquence consensus.

Bien que l'on dispose maintenant de nombreuses données, obtenues in vitro et en système acellulaire, sur la liaison peptide homéoboîte/ADN, on ignorait jusqu'à présent quelles en étaient les conséquences sur les fonctions cellulaires. On ignorait même si les peptides homéoboîte étaient susceptibles de posséder une activité propre, ou si leur rôle se bornait simplement à permettre la liaison homéoprotéine/ADN.

Or, en étudiant l'action de peptides homéoboîte synthétiques sur des cultures cellulaires, les Inventeurs ont découvert des propriétés surprenantes desdits peptides, propriétés qui n'avaient jamais été soupçonnées jusqu'alors.

Ils ont en effet constaté que les peptides homéoboîtes synthétiques, lorsqu'ils sont additionnés à des cellules nerveuses en culture, pénétrent dans toutes les cellules de la culture et que l'entrée des peptides dans les neurones est suivie d'une accumulation dans le noyau. Cette accumulation est non seulement bloquée par préincubation avec un oligonucléotide contenant la séquence consensus ATTA, mais également par préincubation avec des fragments d'ADN double brin non spécifique (c'est-à-dire ne contenant pas la séquence consensus). Les Inventeurs, en analysant ce processus de pénétration, ont démontré l'importance dans celui-ci de la région correspondant à l'hélice 3 + 4 (27 derniers acides aminés du peptide homéboîte).

Ils ont également observé la pénétration de polypeptides comprenant ce peptide homéoboîte.

Ils ont également observé ce phénomène, bien qu'à un degré moindre, dans des cultures de cellules autres que les cellules nerveuses.

Les Inventeurs ont en outre montré que l'accumulation de peptides homéoboîtes dans le noyau était accompagnée d'une croissance et d'une différenciation cellulaire intense.

Ces propriétés des peptides homéoboîtes, démontrées par les Inventeurs, permettent leur utilisation pour l'obtention de nouveaux médicaments, ainsi que leur utilisation *in-vitro* comme agent actif sur des cultures cellulaires.

En effet, il découle des travaux des Inventeurs que les peptides homéoboîtes ou des fragments de ceux-ci, sont à même de fournir de nouveaux facteurs de croissance, en particulier neurotropes, et/ou de nouveaux vecteurs de transport transmembranaires et intracellulaires de molécules actives sur les fonctions cellulaires, en particulier de peptides et d'oligonucléotides.

Or, l'une comme l'autre de ces applications répondent à des besoins actuels. Elles ont fait l'objet de différents travaux dont un bref rappel est fait ci-après.
- L'intérêt des vecteurs de transport intracellulaires de peptides, ou d'oligonucléotides, est apparu consécutivement à la démonstration que certains peptides et oligonucléotides, en se fixant spécifiquement sur certaines régions d'ADN, étaient susceptibles d'agir sur les fonctions cellulaires (par exemple, protéines activant ou réprimant l'expression d'un gène, oligonucléotides anti-sens, etc...).

Toutefois, une exploitation effective, en particulier dans un but thérapeutique, des propriétés de ces molécules, implique de les faire parvenir à pied d'oeuvre en leur faisant franchir les nombreuses barrières séparant le milieu extracellulaire de l'ADN, et en particulier la membrane cytoplasmique. Or, très souvent, ces molécules, de par leur charge, et leur masse moléculaire élevée, ne peuvent pas franchir d'elles mêmes cette barrière. Différentes solutions à ce problème ont été proposées ; certaines d'entre elles, concernant des séquences oligonucléotidiques sont par exemple citées en introduction dans la publication de LEMAITRE et al. [Proc. Natl. Acad. Sci. USA 84, 648-652(1987)]. Ces auteurs proposent quant à eux une approche consistant à lier de façon covalente une séquence oligonucléotidique complémentaire d'une séquence d'ARN du virus de la stomatite vésiculaire (VSV), à un polymère de (L-lysine).

Le conjugué obtenu pénètre dans les cellules et inhibe spécifiquement la synthèse des protéines du VSV dans les cellules infectées.

Ces résultats montrent l'intérêt de l'association entre une macromolécule active et un vecteur de transport de ladite macromolécule. Il est donc particulièrement souhaitable de rechercher de nouveaux vecteurs.
- En ce qui concerne les facteurs de croissance actifs sur la survie et la différenciation des neurones, on n'en connaît actuellement qu'un petit nombre ; le premier qui a été mis en évidence est le Nerve Growth Factor (NGF). L'action du NGF s'exerce essentiellement sur les neurones sensoriels et les neurones du système nerveux sympathique ; une action sur certaines cellules du système nerveux central et du système immunitaire a également été mise en évidence. L'activité neurotropique du NGF est portée par une sous-unité (sous-unité β) de 118 acides aminés.

D'autres substances à action neurotrope ont également été décrites : ce sont, par exemple, le Ciliary Neurotropic Factor (CNTF) [LIN et al, SCIENCE, 246, 1023-1026, (1989) ; STOCKLI et al. NATURE, 342, 920-923, (1989)], le Brain Derived Neurotropic Factor (BDNF) [LEIBROCK et al., NATURE, 341, 149-152, (1989)] , le Glial Derived Nexin (GDN), composant de la matrice extracellulaire [GLOOR et al., CELL, 47, 687-693, (1986)],.

Des facteurs de croissance plus ubiquitaires comme le Fibroblast Growth Factor (FGF) [PARK et HOLLENBERG, DEV. BIOL., 134, 201-205, (1989)] ou l'Epidermial Growth Factor (EGF) [MORRISON et al., SCIENCE, 238, 72-74, (1987)] ont également une action neurotrope.

Le mécanisme d'action de ces facteurs est actuellement mal connu. Il a été montré que le NGF pénètre dans les neurones par l'intermédiaire d'un récepteur spécifique, qui est une glycoprotéine phosphorylée [CHAO et al, Science, 232, 518 (1986)]. A l'intérieur de la cellule nerveuse, le NGF stimule la synthèse d'ARN, par l'intermédiaire d'un second messager.

De nombreuses expérimentations montrent l'intérêt thérapeutique potentiel des facteurs de croissance neurotropes.

L'utilisation du NGF a par exemple été suggérée dans la maladie d'Alzheimer. Il a, en effet, été montré que le NGF permet d'augmenter l'activité choline acétyltransférase des neurones cholinergiques, et de prévenir leur dégénérescence [MOBLEY et al, Science, 229, 284 (1984)], [KROMER, Science, 225, 214, (1987)]. Or il est connu que la maladie d'Alzheimer est associée à une dégénérescence des neurones cholinergiques et à une diminution de l'activité choline acétyltransférase.

Des expérimentations réalisées sur des rats adultes chez lesquels la voie cholinergique reliant hippocampe et septum avait été au préalable détruite (ce qui entraîne une dégénérescence des neurones septaux), ont montré que l'injection intra-ventriculaire de NGF permet la survie des neurones septaux ainsi que la restauration d'une activité normale choline acétyltransférase [WILL et HEFTI, Behav, Brain. Res., 17,17 (1985)]. L'utilisation des facteurs de croissance neurotropes a également été envisagée dans le cas de la maladie de Parkinson, liée à une dégénérescence des neurones dopaminergiques.

Une autre approche du traitement des maladies associées à une dégénérescence neuronale a été récemment proposée et semble promise, dans un proche avenir, à un développement important : il s'agit des greffes intracérébrales de cellules pouvant suppléer aux fonctions neuronales déficientes; l'utilisation de neurones foetaux [LINDVALL et al., SCIENCE, 247, 574-577, (1990)] ou de lignées cellulaires transformées [HORELLOU et al., EUR. J. NEUROSCI., 2, 116-119, (1990)] a ainsi été suggérée.

Récemment des cellules transformées produisant un NGF recombinant ont été implantées dans le cerveau de rats, en même temps que des neurones cholinergiques d'origine foetale. Il a été constaté que, dans ces conditions, la survie des neurones greffés, ainsi que la néogénèse de fibres nerveuses étaient considérablement augmentées [ERNFORS et al, Proc. Natl. Acad. Sci. USA, 86, 4756, (1989)].

Ces travaux montrent donc que les facteurs de croissance neurotropes peuvent trouver des applications particulièrement intéressantes dans le traitement des troubles résultant de lésions ou de dégénérescence neuronale.

Une restriction à l'utilisation des facteurs de croissance neurotropes est toutefois constituée par le petit nombre de facteurs de croissance actuellement connus, ainsi que par leur spécificité d'action relativement étroite, limitée à certains types de neurones. En outre, la plupart de ces facteurs de croissance ne peuvent pas, actuellement, être obtenus en quantité suffisante pour une utilisation thérapeutique.

Il serait donc particulièrement souhaitable de disposer de facteurs de croissance neurotropes qui ne présentent pas les inconvénients qui viennent d'être mentionnés.

La présente Invention, par la démonstration des propriétés inattendues des peptides homéoboîtes, propose une réponse nouvelle à l'un comme l'autre des deux problèmes qui viennent d'être exposés ci-dessus.

La présente Invention a pour objet un polypeptide choisi dans le groupe constitué par les peptides homéoboîtes et les peptides comprenant la séquence correspondant à l'hélice 3 de ceux-ci, pour l'utilisation comme médicament.

Au sens de la présente Invention, on entend par peptide homéoboîte, tout peptide appartenant aux familles définies plus haut, et en particulier toute séquence d'acides aminés présentant avec le peptide pAntp de la Drosophile, une homologie qui permet la fixation de ce peptide à une double hélice d'ADN.

Selon un mode de réalisation préféré de la présente Invention, le peptide homéoboîte est le peptide pAntp.

Selon un mode de réalisation préféré de la présente Invention, le peptide homéoboîte ou son fragment, tels que définis plus haut sont liés à une autre séquence peptidique.

Selon un autre mode de réalisation préféré de la présente Invention, le peptide homéoboîte ou son fragment, sont liés à une séquence nucléotidique.

Les peptides homéoboîtes, ou des fragments de ceux-ci, ainsi que leur produits de fusion avec une autre séquence polypeptidique peuvent être facilement obtenus par des procédés connus en eux-mêmes, par exemple par synthèse peptidique, ou bien par génie génétique. Les produits de fusion d'un peptide homéoboîte et d'une séquence oligonucléotidique peuvent être obtenus par exemple par la technique décrite par LEMAITRE et al.[Proc. Natl. Acad. Sci. USA, 84, 648-652(1987)].

Les propriétés de pénétration intracellulaire des peptides homéoboîtes et de leurs fragments permettent leur utilisation pour introduire dans les cellules des molécules actives sur les fonctions cellulaires, en particulier d'autres peptides, ou des séquences nucléotidiques dotées de propriétés pharmacologiques.

On pourra utiliser un peptide homéoboîte ou un fragment de peptide homéoboîte contenant l'hélice 3, qui reconnaît indépendamment de la séquence, la structure de la molécule d'ADN (sillon large) ; la liaison à une séquence spécifique d'ADN ou d'ARN est alors assurée par le peptide ou l'oligonucléotide lié au peptide homéoboîte ou au fragment de peptide homéoboîte.

Conformément à l'Invention, les polypeptides comprenant un peptide homéoboîte ou un fragment de celui-ci sont également utilisables comme facteurs de croissance, en particulier neurotropes, aussi bien *in-vivo* qu'*in-vitro*.

Contrairement aux facteurs de croissance neurotropes connus dans l'art antérieur, les peptides homéoboîtes sont actifs sur un grand nombre de types de neurones. Les Inventeurs ont en particulier observé l'activité du pAntp sur des cellules nerveuses préparées à partir de diverses régions du système nerveux central embryonnaire, en particulier la moelle épinière, le rhombencéphale, le mésencéphale ventral, le tectum et le cortex. L'activité sur les cellules corticales est particulièrement surprenante, car à ce jour aucune expression d'homéoprotéines n'a été décelée dans cette région du cerveau.

Le large spectre d'action des peptides homéoboîte en fait des agents pharmacologiques d'un grand intérêt, particulièrement dans le traitement des lésions ou dégénérescences neuronales. Ils peuvent également être utilisés dans le domaine des greffes intracérébrales de neurones, qui sont appelées à se développer et pour lesquelles il est indispensable d'assurer la survie ainsi que le développement le plus rapide et le plus étendu en volume du greffon cellulaire.

Ceci peut être réalisé, par exemple, en préincubant les neurones à greffer avec un facteur de croissance conforme à l'Invention, ou bien en réalisant une greffe conjointe des cellules embryonnaires avec des cellules transformées, capable de synthétiser et de secréter les peptides homéoboîte, ou des peptides de fusion contenant une séquence homéoboîte.

La présente Invention a également pour objet un procédé de traitement in vitro de neurones destinés à la greffe, lequel procédé est caractérisé en ce que lesdits neurones sont incubés en présence d'au moins un peptide homéoboîte, éventuellement lié à une autre séquence peptidique ou oligonucléotidique.

La présente Invention a en outre pour objet une composition cellulaire utilisable dans les techniques de greffe de neurones, laquelle composition est caractérisée en ce qu'elle contient une association des neurones que l'on souhaite greffer et de cellules transformées aptes à synthétiser et secréter un peptide homéoboîte.

Les Inventeurs ont également étudié l'action des peptides homéoboîtes non seulement en cultures cellulaires, mais également sur des organismes pluricellaires. Ils ont aussi constaté que grâce à la rapidité de pénétration des peptides homéoboîtes, ceux-ci entraient de façon localisée, dans les cellules adjacentes au point d'injection.

Cette propriété offre l'avantage dans le cadre d'une utilisation sur un organisme vivant, de permettre d'appliquer, si on le souhaite, un traitement très précis, localisé, à un groupe de cellules.

La présente Invention sera mieux comprise à l'aide du complément de description qui va suivre, qui se réfère à des exemples démontrant l'activité des peptides homéoboîtes.

Il va de soi toutefois que ces exemples sont donnés uniquement à titre d'illustration de l'objet de l'Invention dont ils ne constituent en aucune manière une limitation.

### I) OBTENTION DE FACTEURS DE CROISSANCE CONFORMES A L'INVENTION.

### EXEMPLE I : Obtention d'un peptide pAntp

La séquence codant pour l'homéodomaine du gène Antennapedia de la drosophile a été synthétisée en utilisant la technique de la PCR, à partir du plasmide p903G qui contient, entre ses sites BamH1 et PVUII, un fragment de 600bp d'ADNc de l'Antp (GARBER et al., 1983). Les deux amorces dont la séquence est indiquée ci-dessous sont utilisées. La première amorce : (5'GGGGGAATTCCATATGCGCAAACGCGCAAG 3') contient un site de restriction NdeI en amont du codon d'initiation, et la deuxième amorce : (5'GGGGAAGCTTGGATCCTCAGTTCTCCTTCTTCCACTTCAT 3') contient un codon de terminaison suivi par un site BamH1. Un plasmide, dénommé pAH1 a été formé par ligation du fragment NdeI-BamH1 de 220 bp obtenu par PCR, au plasmide pET3a (ROSENBERG et al., 1987). Le polypeptide a ensuite été exprimé dans *E. Coli* BL 21 (Lys S). Les cellules transformées ont été cultivées à 37 °C dans du milieu LB en présence d'ampicilline et de chloramphénicol (100 µg chacun), jusqu'à une densité optique DO₆₀₀ = 1,2. Après une induction de 5 heures en présence de 1 mM d'IPTG, les cellules ont été recueillies par centrifugation (16000 g, 15 min), lavées trois fois au tampon phosphate 50 mM, pH 7,5, NaCl 400 mM, EDTA 2 mM, PMSF 1 mM, DTT 1 mM (tampon A) puis soniquées.

Après centrifugation (16000 g, 15 min) le surnageant a été précipité avec du sulfate de streptomycine (20 mg/ml) sous agitation douce pendant 15 minutes à température de la pièce, puis centrifugé (16000 g, 15 min). Le surnageant a été directement chargé sur une colonne S-SEPHAROSE Fast Flow (PHARMACIA), au préalable équilibrée avec le tampon A. La colonne a été ensuite lavée avec une grande quantité de tampon phosphate 50 mM pH 7,5, NaCl 0,5 M et le peptide pAntp a été élué par application d'un gradient de NaCl (0,5 à 1 M). Le peptide élué (3 mg/l de culture) a été ensuite dialysé pendant 24 heures contre du tampon phosphate 50 mM pH 7,5, NaCl 150 mM.

Les séquences du segment d'ADN amplifié et du peptide ont été déterminées (APPLIED BIOSYSTEM 477) et il a été vérifié qu'elles correspondaient à celles de l'homéoboîte d'Antennapedia. L'analyse du peptide par électrophorèse sur gel en présence de SDS a démontré la présence d'une seule bande du poids moléculaire correspondant à celui de l'homéopeptide d'Antennapedia.

La séquence du peptide obtenu est la suivante :

(I)
NH2Arg Lys Arg Gly Arg Gln Thr Tyr Thr Arg Tyr Gln Thr
Leu Glu Leu Glu Lys Glu Phe His Phe Asn Arg Tyr Leu Thr
Arg Arg Arg Arg Ile Glu Ile Ala His Ala Leu Cys Leu Thr
Glu Arg Gln Ile Lys Ile Trp Phe Gln Asn Arg Arg Met Lys
Trp Lys Lys Glu AsnCOOH

Il a en outre été vérifié, par retardement sur gel du peptide préincubé avec un duplex oligonucléotidique correspondant au site de liaison d'une protéine homéoboîte du type Antennapedia, et obtenu à partir du promoteur Hoxl.3 [Hox1.3p, (ODENWALD et al., GENES AND DM., 3, 158, 1989)] : 3' CAGAGCACGTGATTACCCCCTCAACC 5' 5' GTCTCGTGCACTAATGGGGGAGTTGG 3' que le peptide reconnait in vitro la séquence consensus de fixation des protéines de type Antennapedia.

### II) DEMONSTRATION DE L'ACTIVITE DES FACTEURS DE CROISSANCE CONFORMES A L'INVENTION

### Exemple 2 : Démonstration de l'effet biologique du peptide pAntp sur des neurones en culture

Les neurones prélevés dans différentes régions du cerveau (mésencéphale , corde spinale, cortex) d'embryons (E14 à E16) de rat sont mis en culture dans un milieu défini (CHAMAK and PROCHIANTZ, DEVELOPMENT, 106, 483, 1989) permettant la survie des seules cellules neuronales (milieu CDM).

Le peptide pAntp est renaturé par une incubation de 10 min à 60 °C en présence de dithiothréitol (0,1 mM) et de Magnésium (10 mM) dans un tampon phosphate pH 7,2 isotonique contenant 33 mM de D-Glucose. Le peptide est rajouté aux cellules nerveuses à une concentration de 9 µg/ml (soit 1,3 µM).

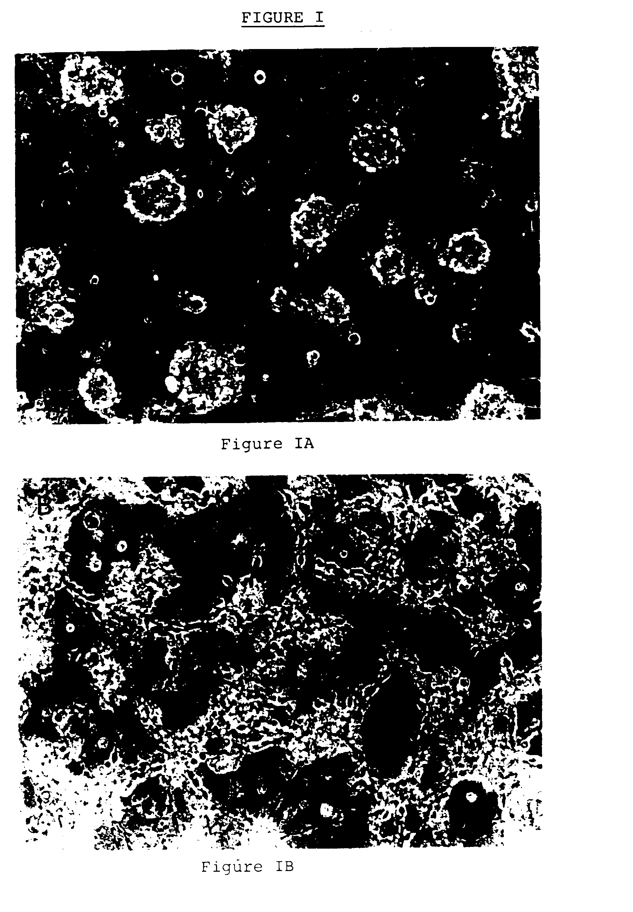

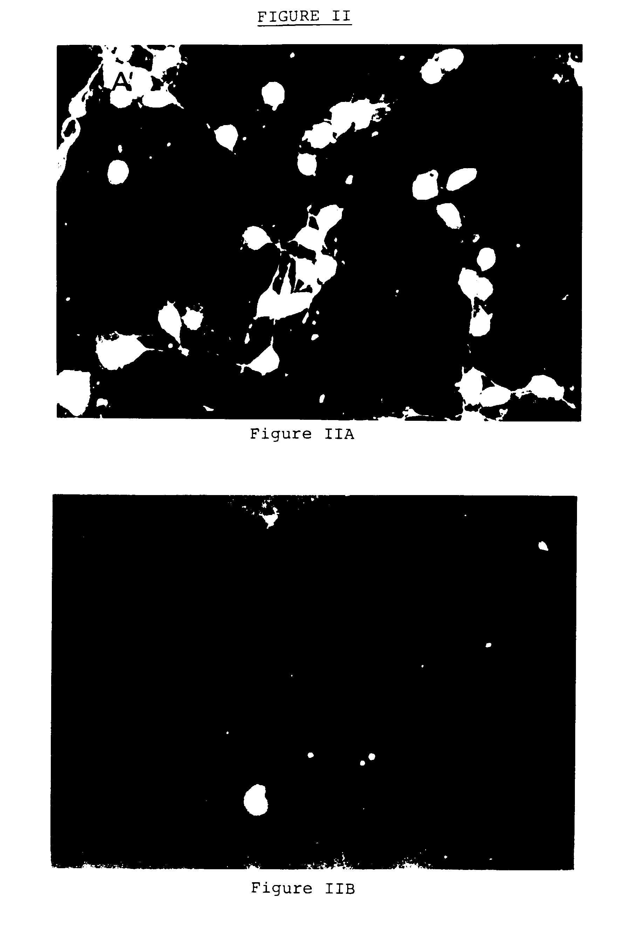

Les effets observés après l'addition du peptide sont illustrés dans la figure 1 (A et B). La figure 1A montre l'aspect de cellules témoins cultivées pendant 24 heures en l'absence du peptide. La figure 1B montre l'aspect des cellules cultivées pendant 24 heures en présence du peptide. On note, dès 24h après l'addition, une considérable augmentation de la croissance neuritique dans les cellules cultivées en présence du peptide pAntp.

Les mêmes résultats sur la croissance cellulaire ont en outre été observés lors d'expériences dans lesquelles les cellules étaient préincubées avec peptide pAntp, puis remises en culture pendant 24 heures dans du milieu CDM dépourvu du peptide. Une incubation de 30 minutes en présence de pAntp permet déjà d'observer les effets décrits ci-dessus ; ces effets sont maximaux après une incubation de 2 heures, et ne sont pas augmentés par un prolongement du temps de préincubation.

La préincubation du peptide homéoboîte avec la séquence consensus de fixation obtenue à partir du promoteur Hox1.3, ou bien avec un mélange de fragments (de séquence aléatoire) d'ADN en double hélice, bloque son effet biologique.

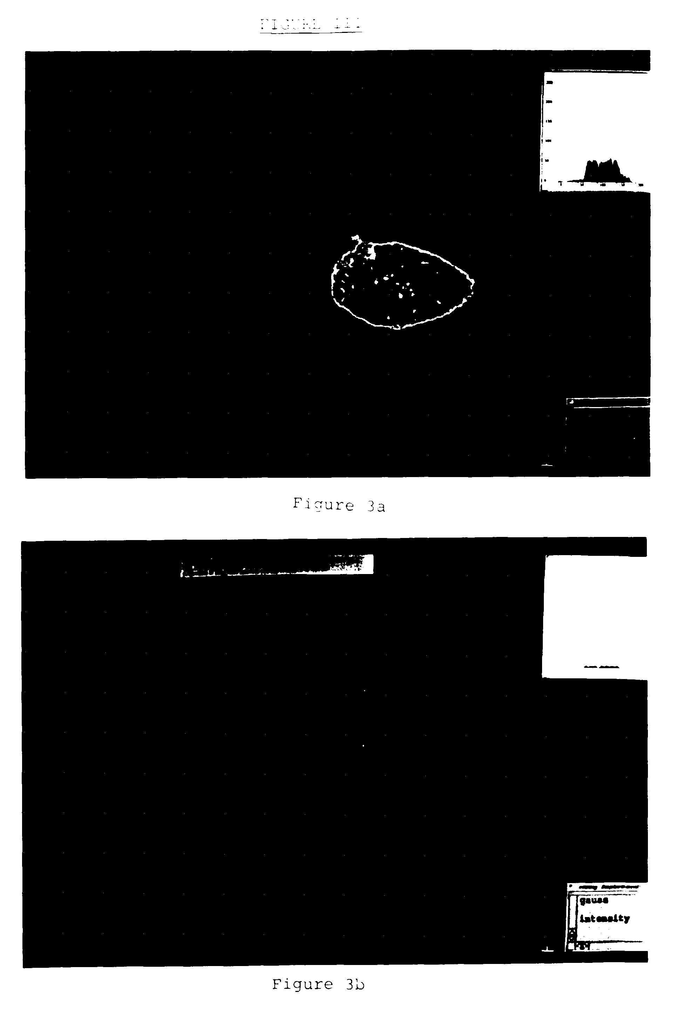

Il a en outre été montré, en utilisant un peptide marqué à la fluorescéine, que le peptide homéoboîte est rapidement (moins de 2 heures) capturé par tous les neurones, puis transporté dans le noyau (figure 2A), et que ce transport est bloqué après préincubation avec l'oligonucléotide consensus obtenu à partir du promoteur Hox1.3 ou bien avec mélange de fragments (de séquence aléatoire) d'ADN en double hélice (Figure 2B).

Une comparaison entre l'entrée et la répartition intracellulaire du peptide pAntp et de la polyornithine a également été effectuée.

Les cellules ont été incubées pendant 1 heure en présence de pAntp ou de polyornithine, marqués à la fluorescéine.

Les résultats sont illustrés par la figure 3 qui montre que le pAntp marqué (3A) est retrouvé principalement dans le nucléoplasme, contrairement à la polyornithine présente dans le soma et les nucléoles (3B).

### Exemple 3 : Pénétration du peptide pAntp dans des fibroblastes en culture.

Les fibroblastes sont obtenus à partir de peau d'embryons de rats, dissociés mécaniquement et trypsinisés ; les cellules sont cultivées sur des boîtes de verre recouvertes de poly-DL-ornithine (SIGMA, PM 40.000, 15 µg/ml), dans du milieu de culture DMEM-F12 (GIPCO-PRL), et en présence de 10% de sérum de veau foetal. Après 24 heures de cultures, les cellules sont incubées pendant une heure en présence de peptide pAntp marqués à la fluorescéine. Le peptide pAntp marqué pénètre dans les fibroblastes, mais est présent dans le noyau en moindre quantité que dans les cellules nerveuses.

### Exemple 4 : Pénétration du peptide pAntp dans des cellules embryonnaires in-vivo.

0,2 µl de solution (1 µg/µl) du peptide pAntp marqué à la fluorescéine sont injectés dans le mésencéphale d'un embryon de caille de 2 jours. Après 4 heures d'incubation *in-ovo*, l'embryon est extrait de l'oeuf, et des coupes du mésencéphale sont effectuées. L'observation de ces coupes au microscope montre que le marquage fluorescent est localisé au noyau des cellules situées à proximité immédiate du point d'injection.

Ainsi que cela ressort de ce qui précède, la présente invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Polypeptide, choisi dans le groupe constitué par les peptides homéoboîtes et les peptides comprenant la séquence de l'hélice 3 d'un peptide homéoboîte, pour l'utilisation comme médicament.

2. Polypeptide selon la Revendication 1, caractérisé en ce que ledit peptide homéoboîte est le peptide pAntp.

3. Polypeptide selon l'une quelconque des Revendications 1 ou 2, caractérisé en ce qu'il est lié à une autre séquence polypeptidique.

4. Polypeptide selon l'une quelconque des Revendications 1 ou 2, caractérisé en ce qu'il est lié à une séquence oligonucléotidique.

5. Polypeptide selon l'une quelconque des Revendications 1 ou 2, pour l'utilisation comme facteur de croissance cellulaire, en particulier neurotrope.

6. Polypeptide selon l'une quelconque des Revendications 1 ou 2, pour l'utilisation comme vecteur de transport intracellulaire de molécules actives sur les fonctions cellulaires.

7. Utilisation d'un polypeptide tel que défini dans les Revendications 1 à 4, comme agent actif sur des cellules en culture.

8. Utilisation selon la Revendication 7, caractérisée en ce que ledit polypeptide est utilisé comme facteur de croissance.

9. Utilisation selon la Revendication 7, caractérisée en ce que ledit polypeptide est utilisé comme vecteur de transport intracellulaire de molécules actives sur les fonctions cellulaires.

10. Utilisation selon l'une quelconque des Revendications 7 à 9, caractérisée en ce que les cellules en culture sont des neurones.

11. Procédé de traitement in vitro de neurones destinés à la greffe, lequel procédé est caractérisé en ce que lesdits neurones sont incubés en présence d'au moins un peptide homéoboîte, éventuellement lié à une autre séquence peptidique.

12. Composition cellulaire utilisable dans les techniques de greffe de neurones, laquelle composition est caractérisée en ce qu'elle contient une association des neurones que l'on souhaite greffer, et de cellules transformées aptes à synthétiser et secréter un peptide homéoboîte ou un peptide de fusion comprenant une séquence homéoboîte.

13. Composition pharmaceutique, caractérisée en ce qu'elle comprend en tant que principe actif, au moins un polypeptide tel que défini dans l'une quelconque des Revendications 1 à 4.

## Patentansprüche

1. Polypeptid, ausgewählt aus der Gruppe, bestehend aus Homöobox-Peptiden und Peptiden, umfassend die Sequenz der Helix 3 eines Homöobox-Peptids zur Verwendung als Medikament.

2. Polypeptid nach Anspruch 1, dadurch **gekenn-zeichnet**, daß das Homöobox-Peptid das Peptid pAntp ist.

3. Polypeptid nach einem der Ansprüche 1 oder 2, dadurch **gekennzeichnet**, daß es an eine andere Polypeptidsequenz gebunden ist.

4. Polypeptid nach einem der Ansprüche 1 oder 2, dadurch **gekennzeichnet**, daß es an eine Oligonucleotid- Sequenz gebunden ist.

5. Polypeptid nach einem der Ansprüche 1 oder 2 zur Verwendung als zellulärer, insbesondere als neurotroper Wachstumsfaktor.

6. Polypeptid nach einem der Ansprüche 1 oder 2 zur Verwendung als intrazellurärer Transportvektor für Moleküle, die auf die Zellfunktionen einwirken.

7. Verwendung eines in den Ansprüchen 1 bis 4 definierten Polypeptids als auf Zellen in Kultur einwirkendes Mittel.

8. Verwendung nach Anspruch 7, dadurch **gekennzeichnet**, daß das Polypeptid als Wachstumsfaktor verwendet wird.

9. Verwendung nach Anspruch 7, dadurch **gekennzeichnet**, daß das Polypeptid als intrazellulärer Transportvektor für auf die Zellfunktionen einwirkende Moleküle verwendet wird.

10. Verwendung nach einem der Ansprüche 7 bis 9, dadurch **gekennzeichnet**, daß die Zellen in Kultur Neuronen sind.

11. Verfahren zur in vitro Behandlung von Neuronen für die Implantation, dadurch **gekennzeichnet**, daß die Neuronen in Gegenwart von mindestens einem Homöobox-Peptid, gegebenenfalls in Bindung an eine andere Peptidsequenz, inkubiert werden.

12. Zelluläre Zusammensetzung zur Verwendung in den Techniken zur Implantation von Neuronen, dadurch **gekennzeichnet**, daß sie eine Assoziation von zu implantierenden Neuronen und transformierten Zellen mit der Eignung zur Synthese und Sekretion eines Homöobox-Peptids oder eines eine Homöobox-Sequenz umfassenden Fusionspeptids enthält.

13. Pharmazeutische Zusammensetzung, dadurch **gekennzeichnet**, daß sie als Wirkstoff mindestens ein Polypeptid, wie in einem der Ansprüche 1 bis 4 definiert, enthält.

## Claims

1. A polypeptide, chosen from the group comprising the homeobox peptides and the peptides comprising the helix 3 sequence of a homeobox peptide, for use as a medicament.

2. A polypeptide according to Claim 1, characterised in that the said homeobox peptide is the peptide pAntp.

3. A polypeptide according to either of Claims 1 or 2, characterised in that it is bonded to another polypeptide sequence.

4. A polypeptide according to either of Claims 1 or 2, characterised in that it is bonded to an oligonucleotide sequence.

5. A polypeptide according to either of Claims 1 or 2, for use as a cellular, in particular a neurotropic growth factor.

6. A polypeptide according to either of Claims 1 or 2, for use as an intracellular transport vector for molecules active on cellular functions.

7. Use of a polypeptide as defined in Claims 1 to 4 as an agent active on cells in culture.

8. Use according to Claim 7, characterised in that the said polypeptide is used as a growth factor.

9. Use according to Claim 7, characterised in that the said polypeptide is used as an intracellular transport vector for molecules active on cellular functions.

10. Use according to any one of Claims 7 to 9, characterised in that the cells in culture are neurones.

11. A method for the in vitro treatment of neurones intended for grafting, the said method being characterised in that the said neurones are incubated in the presence of at least one homeobox peptide, possibly bonded to another peptide sequence.

12. A cellular composition which can be used in neurone grafting techniques, said composition being characterised in that it contains an association of the neurones which it is desired to graft and transformed cells adapted to synthesise and secrete a homeobox peptide or a fusion peptide comprising a homeobox sequence.

13. A pharmaceutical composition, characterised in that it comprises as its active principle at least one polypeptide as defined in any one of Claims 1 to 4.
